# EUROPEAN PATENT APPLICATION

(11) **EP 4 841 648 A2**
(43) Date of publication of application: **18.11.2026**
(21) Application number: 26208586.3
(22) Date of filing: 24.05.2019
(51) Int. Cl.: G16H 50/20

(54) **SIGNAL REPLAY FOR SELECTION OF OPTIMAL DETECTION SETTINGS**

(30) Priority: 28.01.2019 US 201962797383 P
(62) Document number(s) of the earlier application(s) in accordance with Art. 76 EPC: 19176404.2 / 3 686 896
(71) Applicant: BIOTRONIK SE & Co. KG, 12359 Berlin (DE)
(72) Inventor: PETERSON, Jon Nels, Lake Oswego, 97035 (US); WHITTINGTON, R. Hollis, Portland, 97202 (US)
(74) Representative: Biotronik Corporate Services SE

(57) **Abstract**

The present invention relates to a method for providing adjusted parameters of an medical device, comprising the steps of: collecting patient data by means of the medical device, wherein the medical device comprises programmable parameters for affecting a function carried out by the medical device, transmitting said patient data to an external device, conducting an analysis of the transmitted patient data by means of the external device, and determining an optimized parameter setting for the medical device by repeatedly applying an analysis algorithm to stored patient data using a plurality of parameter combinations and selecting a best parameter combination based on the resulting classifications. Furthermore, the invention relates to a corresponding system.

## Description

The present invention relates to a method and a system for providing adjusted parameters of a medical device.

Algorithms designed to detect certain clinical events often have multiple user-selectable parameter settings to allow for individual variance in patient signals. Default settings are often used, however, as real-time evaluation of each combination of settings is both time consuming and difficult to evaluate. For example, QRS detection algorithms may have selectable filtering and threshold parameters for optimal detection of the QRS complex while rejecting T-waves and muscle artifact. Arrhythmia detection algorithm parameters are selected along a spectrum of higher sensitivity versus higher specificity.

According to the prior art, clinicians often manually select from the available parameter set, then evaluate their selection either immediately through real-time data (e.g., for QRS detection), or over time via statistics and stored data (e.g., arrhythmia detection) transmitted from or retrieved from the device.

US 9,629,548 B2 describes a system, data collection method and workflow for predicting HF decompensation, wherein patient data is collected by an implantable device. The data is sent to an external device/data server and analyzed. The physician inputs feedback to the analyzed data. The data analysis is modified according to the physician's feedback.

Typically, algorithms known in the art have multiple parameters available for adjustment, resulting in a large number of parameter combinations. For example, three separate parameters, each with three possible values, result in 27 possible settings. Due to the time required to test each possibility, and the difficulty in evaluating the result, default settings are often used unless there are significant performance issues. There is often a long time period required to optimize settings in the current practice. Settings are adjusted and days or weeks elapse before the impact of those settings is realized. In the absence of remote programming, a subsequent follow up is required in order to correct poorly performing settings and bad adjustments. This also implies a long time lag in order to improve settings during the initial implant and tuning of the device.

Based on the above it is therefore an objective of the present invention to provide a method and a system that allows to efficiently optimize parameter settings for the medical device.

This objective is solved by a method comprising the features of claim 1 and by a system comprising the features of claim 9. Preferred embodiments are stated in the corresponding dependent claims and are described below.

According thereto, a method for providing adjusted parameters of a medical device is disclosed, comprising the steps of:
- collecting patient data by means of the medical device, wherein the medical device comprises programmable parameters for adjusting a function carried out by the medical device,
- transmitting or communicating said patient data to an external device,
- conducting an analysis of the transmitted patient data on the external device, and
- determining an optimized parameter setting for the medical device by repeatedly applying an analysis algorithm to stored patient data using a plurality of parameter combinations and selecting a best parameter combination based on the resulting classifications.

According to an embodiment of the present method, wherein the at least one adjusted programmable parameter is automatically programmed to the medical device. According to an embodiment, at least one adjusted programmable parameter is remotely programmed to the medical device.

According to embodiments of the present invention, the medical device is an implantable medical device as for example a cardiac pacemaker, a spinal cord stimulator, a vagus nerve stimulator, a deep brain stimulator, or the like. The medical device is, according to an embodiment of the invention, a non-implantable medical device, as for instance a medical device as for instance a Holter monitor.

Particularly said proposal can be displayed to the physician via a graphical user interface (GUI), particularly a GUI of the external device.

Thus, the present invention provides an opportunity to replay stored patient data (either on the external device, e.g. a programmer, or in an external data center) through a detection algorithm using all available settings of the parameters. The resulting performance can be presented as a function of settings, thereby allowing the clinician to choose the optimal parameter settings for this patient based on the collected patient data. In the context of the present invention, 'patient data' is understood as information on at least a physiological parameter of a patient, which is measured via an external device or an implantable medical device and converted into a signal. As an example, cardiac monitoring devices typically measure electrocardiogram (ECG) signals, wherein brain diagnostics devices measure electroencephalogram (EEG) signals, devices for volumetric measurements of an organ measure plethysmogram (PG) signals, etc. The present invention is applicable to all kinds of signals / patient data where an analysis of the patient data is desired. In the following the term "signal" is used as synonym for all kinds of possible signals representing a physiological parameter of a patient and measured by a said medical device. "patient data" is used as synonym for any kind of physiological patient parameter which is measured via a said medical device via a signal.

Particularly, according to an embodiment, the external device can be a programming device (also denoted as a programmer), a single computer, or a network comprising several computers. The proposal can be displayed to the physician via a graphical user interface (GUI), particularly a GUI of the external device.

Particularly, in the context of the present invention, the notion "external device" means that the external device is arranged remote from the medical device, or outside the body of the patient when the medical device is an implantable medical device in an implanted state. Furthermore, implanting the implantable medical device into the patient does not form a step of the method according to the present invention, i.e. the method uses an implantable medical device that has been implanted into the patient before carrying out the method according to the present invention.

Particularly, according to an embodiment of the method, said event-detection function is carried out by the medical device by executing an algorithm implemented in the medical device, wherein said algorithm utilizes said programmable parameters to achieve a classification of clinical events such as but not limited to QRS detections or detections of atrial fibrillation, contained in said collected patient data.

According to the present invention, a clinical event would be any event that would have relevance to the medical condition of the patient. This could be events detectable in the signal / patient data. E.g., for ECGs, clinical events could be those relating to the rhythm like arrhythmias. Clinical events can occur in another biological signal that gives information about the body (e.g. PG, impedance signal, evoked compound action potentials (ECAPS), EEGs, Electromyogram (EMG) signals, etc. etc.).

According to an exemplary QRS detection metric, the ECG signal is analyzed via high pass 0.5 Hz, high pass 10Hz, high pass 18 Hz, or high pass 24 Hz filters. According to another example, typical thresholds for signal amplitude detection are set to 0.1mV, 0.2mV, 0.3mV.

The algorithm can be implemented in the medical device in various fashions, and particularly when any of one or several parameters can be selected to alter the algorithm performance, it is relevant to this invention. According to an embodiment of the present invention, the algorithm or updates of the algorithm may be uploaded to the medical device via an external device or a service center.

Furthermore, according to an embodiment of the method, the step of collecting patient data by means of the medical device corresponds to measuring an electrocardiogram (ECG), e.g. a particular ECG strip or group of ECG strips, i.e. said patient data is an ECG measured by means of the medical device.

Furthermore, according to an embodiment of the method, the collected patient data is stored in the external device, particularly for later review. The measured signal can be evaluated by a physician and/or via algorithms for the occurrence of signals indicating a pathophysiology. In case of an ECG, pathophysiologies may be e.g. bradycardia, tachycardia, atrial fibrillation, ectopies, changes in the heart rate, asystolies or the like.

Further, according to an embodiment of the method, conducting said analysis comprises carrying out the same algorithm on the external device and thereby applying the algorithm to the collected patient data for a plurality of different settings of each programmable parameter, wherein for each setting of the parameters the classification of clinical events obtained by the algorithm executed on the external device are stored (e.g. on the external device).

Further, according to an embodiment of the method, a human expert (e.g. physician) or an expert system independently processes the classifications of the clinical events made by the algorithm executed on the external device to adjudicate them as true or false.

Further, according to an embodiment, the method according to the invention comprises the steps:
1. Collecting patient data by means of the medical device, wherein the medical device comprises programmable parameters for adjusting a function carried out by the medical device,
2. Transmitting or communicating said patient data to an external device or data center,
3. Conducting an analysis of the transmitted patient data on the external device or data center, wherein the analysis comprises adjudication of the data as true or false,
4. Identify a 'best combination' of programmable parameters for adjusting a function carried out by the medical device based on step 3
5. Conducting an evaluation of the transmitted patient data using the 'best combination' identified in step 4.

The adjudication is most effective on the implant data and could be made on a programmer or other external device. According to embodiments of the inventive method, the patient data is analyzed and evaluated through multiple iterations. Input from a human expert is not required in the data analysis / evaluation process. However, it is not categorically to be excluded that human expert knowledge can be used for adjusting certain steps of the inventive method, as e.g. for optimization of the algorithms behind the analysis of transmitted patient data in step 3 above.

According to an embodiment, the best combination identified in step 4 is automatically selected as the parameter setting to be applied to the medical device.

Further, according to an embodiment of the method, said automatically computed proposal for adjusting at least one of the parameters, or several of the parameters, or all of the parameters is adapted such that false classifications of clinical events made by the algorithm are reduced when the respective parameter is adjusted according to the proposal.

In this way, the expert (physician) or expert system, with knowledge of the algorithm and its programmable parameters, determines or influences the optimum settings of the programmable parameters of the algorithm. Particularly, the optimum parameter settings that are now patient specific, are presented to the user (e.g. physician) for review and acceptance.

According to a further embodiment of the method, the user (e.g. physician) provides a preference or guidance regarding the type of performance. For instance, a higher sensitivity of the algorithm and a lower specificity, or vice versa. Based on these preferences, the optimization algorithm chooses parameter settings which provide performance matching that preference.

Furthermore, according to an embodiment, an evaluation and adjudication of the patient data / clinical events are performed in real-time by the clinician's programmer forming the external device and said proposal for adjusting one, or several, or all of the parameters is particularly made in real-time during a follow-up. Alternatively, the historical data stored on the programmer or on an external device could be leveraged to improve the optimization further and to adjust to the patient's longer-term health trajectory.

Further, according to an embodiment of the method according to the present invention, said function of the medical device corresponds to one of:
- Detecting of Q, R, and S waves in the ECG, wherein particularly the respective clinical event is the occurrence of a Q, R or S wave,
- Detecting of P waves in the ECG, wherein particularly the respective clinical event is the occurrence of a P wave,
- Detecting of T waves in the ECG, wherein particularly the respective clinical event is the occurrence of a T wave,
- Detecting of ectopic events from the atrium or ventricle, wherein particularly the respective clinical event is the occurrence of a ectopy,
- Detecting of atrial fibrillation in the ECG, wherein particularly the respective clinical event is the occurrence of an atrial fibrillation,
- Detecting of bradycardia in the ECG, wherein particularly the respective clinical event is the occurrence of bradycardia,
- Detecting of an asystole in the ECG, wherein particularly the respective clinical event is the occurrence of an asystole,
- Detecting of a sudden rate drop in the ECG, wherein particularly the respective clinical event is the occurrence of a sudden rate drop,
- Detecting of a high ventricular rate in the ECG, wherein particularly the respective clinical event is the occurrence of a high ventricular rate (e.g. above a pre-defined threshold).

Moreover, according to an embodiment of the inventive method, said parameters of the medical device (2) are adjusted via the external device (3), another external device or an external data center via wireless programming.

A further aspect of the present invention relates to a system for determining parameter settings of a medical device, wherein the system comprises:
- a medical device configured to collect patient data representing at least one physiological signal and comprising programmable parameters,
- an external device configured to receive stored patient data, execute an analysis algorithm on the stored patient data, repeatedly apply the analysis algorithm for a plurality of different parameter combinations, determine classification results of at least one clinical event for the respective parameter combinations, compare the classification results, and select a parameter combination as an optimized parameter setting for the medical device.

Particularly, the implantable medical device can be an implantable monitoring device or an implantable cardiac pacemaker or an implantable cardioverter defibrillator.

Further, according to an embodiment of the system according to the present invention, the medical device is configured to carry out said function by executing an algorithm implemented in the medical device, wherein said algorithm utilizes said programmable parameters to achieve a classification of clinical events contained in said patient data.

Particularly, according to an embodiment of the system, the collected patient data is an ECG of the patient, particularly a real-time ECG.

Furthermore, particularly, the system is configured to store the collected patient data (e.g. ECG) in the external device, particularly for later review.

Further, according to an embodiment of the system, the external device is configured to conduct said analysis by executing the same algorithm on the external device and apply the algorithm to the collected patient data for a plurality of different settings of each parameter, wherein the external device is further configured to store for each setting of the parameters the classifications of clinical events obtained by the algorithm executed on the external device.

Further, according to an embodiment of the system, the system is configured to process, as an input, adjudications by a human expert (e.g. physician) or an expert system whether the respective classification made by the algorithm executed on the external device is true or false.

Further, according to an embodiment of the system, the system is configured to adapt said automatically computed proposal for adjusting at least one of said parameters, or several of said parameters, or all of said parameters, such that false classifications of clinical events made by the algorithm are reduced when the respective parameter is adjusted according to the proposal.

Particularly, according to an embodiment of the system, said function corresponds to one of (see also above):
- Detecting of P, Q, R, S, and T waves in the signal,
- Detecting of atrial fibrillation in the signal,
- Detecting of bradycardia in the signal,
- Detecting of an asystole in the signal,
- Detecting of a high ventricular rate in the signal
- Detecting a suddent rate drop in the signal.

In the following embodiments as well as further features and advantages of the present invention are described with reference to the Figures, wherein
- Fig. 1: a schematic illustration of an embodiment of a system/method according to the present invention;
- Fig. 2: shows an example of an optimization matrix used to pick ideal sensing parameter settings according to an embodiment of the method of the present invention;
- Fig. 3: shows an example of arrhythmia assessment across two parameters according to an embodiment of the method of the present invention;
- Fig. 4: shows multiple snapshot adjudication and selection for optimization according to an embodiment of the method according to the present invention.
- Fig. 5: an exemplary matrix for classification results of a single signal episode ('snapshot')
- Fig. 6: an exemplary matrix for classification results of multiple signal episodes ('snapshots')

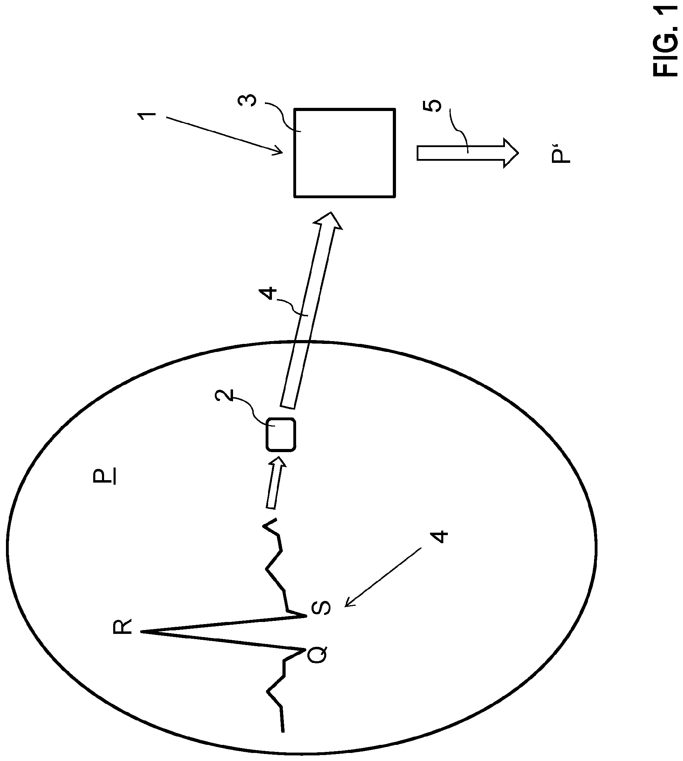

Fig. 1 shows a schematic illustration of an embodiment of a system 1 / method according to the present invention. According thereto, the system 1 comprises an medical device 2 configured to collect patient data 4 of a patient P wherein the medical device 2 is configured to perform a function and comprises programmable parameters for affecting said function. Further, the system 1 comprises an external device 3, wherein the medical device 2 is configured to transmit said collected patient data 4 to the external device 3, wherein the external device 3 is further configured to conduct an analysis of the transmitted patient data 4, and wherein the external device 3 is configured to determine and provide an optimized parameter setting 5 e.g. to a physician P' for adjusting at least one of said parameters, or several of said parameters, or all of said parameters using said analysis.

The external device 3 may further be configured to repeatedly apply an analysis algorithm to stored patient data 4 using a plurality of parameter combinations, determine classification results for the respective parameter combinations, compare the classification results and select an optimized parameter setting for the medical device 2.

Particularly, the external device 3 can be a programmer that is operable to program said programmable parameters of the medical device 2. The medical device 2 can be a monitoring device that is e.g. configured to monitor a signal 4 of the patient P. Alternatively the medical device 2 can be a cardiac pacemaker or a cardioverter defibrillator.

Particularly, according to an embodiment, for QRS detection, a real-time electrocardiogram (ECG) is captured by the external device 3 as said patient data 4, wherein the external device 3 can be formed by a physician's programmer. The data 4 can be either directly received from the implant 2 or can be stored in the implant before. Particularly, a QRS detection algorithm that would normally be applied by the device 2 is applied to the captured ECG 4 by the external device 3, using e.g. all variations of the available parameter set. Particularly, a performance metric such as a Receiver Operating Curve (sensitivity versus 1-specificity) allows the clinician P' to see which parameter set(s) would be most appropriate for this patient P. The clinician P' can select a candidate from this ROC plot directly, or may examine the ECG 4 with detection annotations before selecting a candidate.

Since the algorithm for conducting data analysis is now running on the external device 3 (e.g. programmer), which does not have the processor and battery limitations of the implant 2, a much wider parameter space can be explored.

Particularly, a human expert (e.g. clinician / physician) P' or an expert system independently processes the classifications of the events made by the algorithm executed on the external device 3 to adjudicate them as true or false. Said automatically computed proposal is then adapted such that false classifications of clinical events made by the algorithm are reduced or true classifications are increased when the respective parameter is adjusted according to the proposal.

Apart from QRS detection described above, the function/algorithm may also be configured to detect one of: atrial fibrillation, bradycardia, asystole, sudden rate drop, or a high ventricular rate.

Particularly, according to an embodiment of the invention, when the function of the implantable device 2 relates to detecting bradycardia, a false snapshot can be re-evaluated with different bradycardia settings. These settings can be applied to other non-bradycardia snapshots of the measured ECG 4, to ensure that they are not falsely detected. A more sophisticated approach would be to evaluate both bradycardia and QRS detection for optimal settings of QRS detection in the context of bradycardia. For instance, various QRS detection settings can be applied to find the settings where a "false" bradycardia snapshot is no longer detected as false. The user P' can adjudicate the snapshot or adjudicate specific events in order to provide a gold standard for grading the parameters.

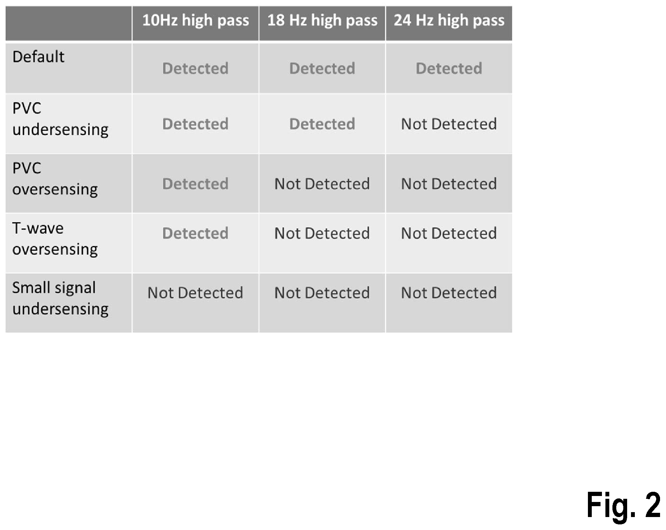

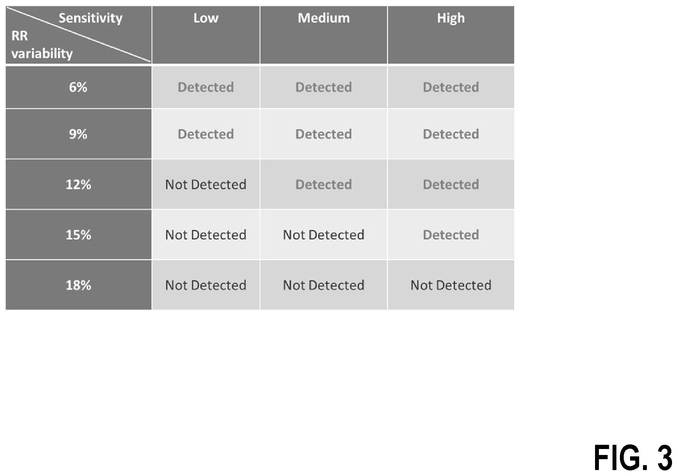

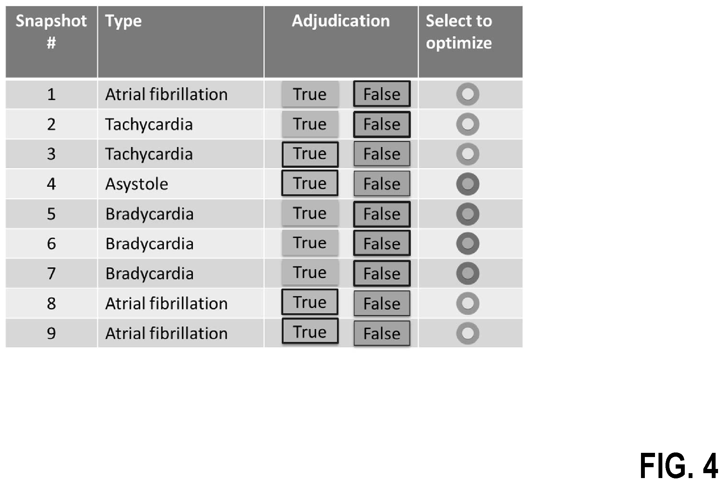

Particularly, the present invention allows for improving performance by leveraging the parameter space of existing algorithms. The human or machine experts P' are presented with performance data, so that they can use their domain experience to choose the best parameters for their patient P without going through the drudgery and time of manually selecting each parameter. This minimizes the need for customer support of patients P. By providing a graphical matrix of parameter settings (cf. e.g. Figs. 2 and Fig. 3), along with performance at those settings, the user P' can at a glance select settings of the programmable parameters that perform to their expectations. In addition, by selecting multiple snapshots, the algorithm can provide a pooled performance metric that has a more generalized behavior applicable to the patient's signals (cardiac rhythm or ECG morphology (Fig. 4), where the medical device measures cardiac signals). Snapshots can be adjudicated based on review and then selected for optimization to achieve the performance desired for a given subset of snapshots. This achieves a personalized medicine outcome with appropriate settings for either sensing settings or arrhythmia settings based on individual patients P.

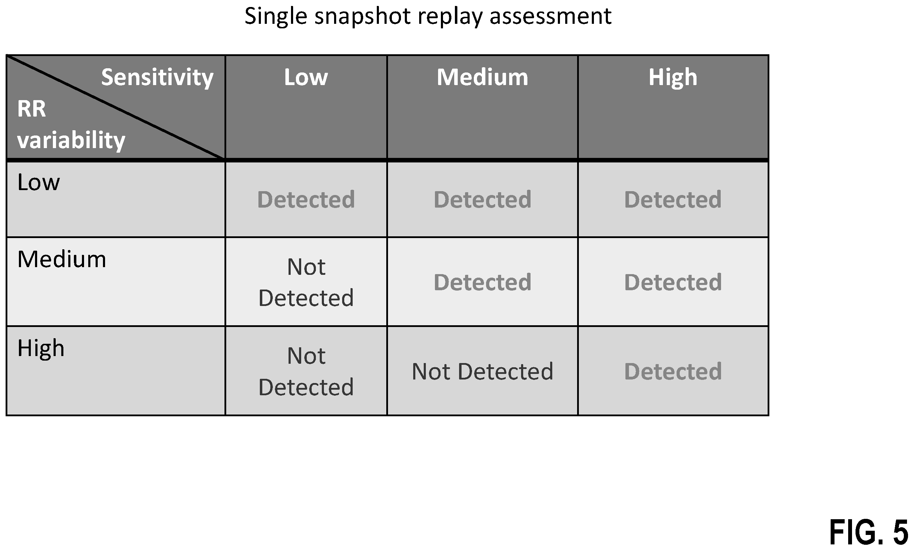

Fig. 5 shows an example for classification of a single signal episode ('snapshot') of an ECG according to an embodiment of the invention. The adjustable parameters are "sensitivity", which is technically defined by the formula (true positives)/(true positives + false negatives) and RR variability, which measures the variation of time intervals between successive R-peaks in an ECG for a given time window. The matrix has three columns which stand for sensitivity in three levels "low", "medium" and "high", and three rows which stand for RR variability also in three levels "low", "medium" and "high". The cells of the matrix contain and for each possible combination of the two parameter and levels exemplary classification results as "detected" or "not detected" for the detection of a clinical event in the snapshot. In this case, a clinical event was detected for 6 of the 9 combinations, while for 3 of the 9 combinations, no clinical event was detected.

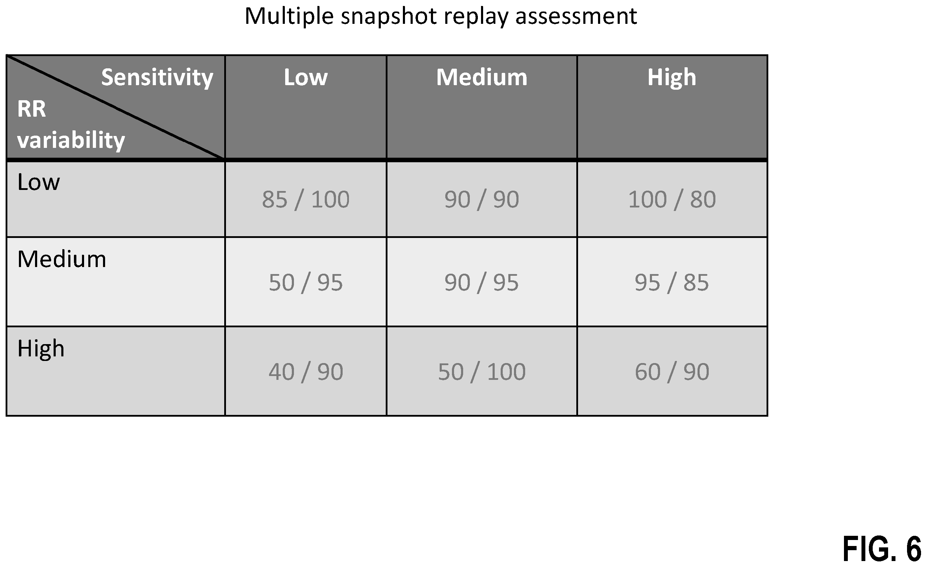

Fig. 6 shows an example for classification according to Fig. 5 for multiple snapshots. The first number of each matrix cell refers to the sensitivity in percent %, the second number to the precision (Positive Prediction Value PPV, defined by the formula (true positives) / (true positives + false positives)) in percent. The user may choose the desired parameter settings according to the indicated sensitivity vs. precision.

## Claims

1. A method for determining parameter settings of a medical device, the method comprising:
receiving stored patient data representing at least one physiological signal collected by a medical device;
executing, on an external computing device, an analysis algorithm on the stored patient data;
**characterized in that** the method further comprises:
repeatedly applying the analysis algorithm to the stored patient data for a plurality of different combinations of programmable parameters of the medical device;
determining, for each of said combinations, a corresponding classification result of at least one clinical event;
comparing the classification results obtained for the plurality of parameter combinations; and
selecting a parameter combination based on said comparison as an optimized parameter setting for the medical device.

2. The method according to claim 1, wherein the analysis algorithm corresponds to an event-detection algorithm implemented in the medical device.

3. The method according to claim 1 or 2, wherein the clinical event comprises at least one of: QRS detection, P-wave detection, T-wave detection, atrial fibrillation detection, bradycardia detection, asystole detection, sudden rate drop detection, and high ventricular rate detection.

4. The method according to one of the preceding claims, wherein the classification results are adjudicated as true or false.

5. The method according to claim 4, wherein the optimized parameter setting is selected such that false classifications are reduced.

6. The method according to one of the preceding claims, wherein the comparison comprises determining a performance metric for each parameter combination.

7. The method according to claim 6, wherein the performance metric comprises sensitivity and/or precision.

8. The method according to one of the preceding claims, wherein the optimized parameter setting is automatically programmed to the medical device.

9. A system (1) for determining parameter settings of a medical device (2), comprising:
an medical device (2) configured to collect patient data (4) representing at least one physiological signal and comprising programmable parameters, and
an external device (3) configured to:
receive stored patient data (4) representing the at least one physiological signal collected by the medical device (2);
execute an analysis algorithm on the stored patient data (4);
repeatedly apply the analysis algorithm to the stored patient data (4) for a plurality of different combinations of programmable parameters of the medical device (2);
determine, for each of said combinations, a corresponding classification result of at least one clinical event;
compare the classification results obtained for the plurality of parameter combinations; and
select a parameter combination based on said comparison as an optimized parameter setting for the medical device (2).

10. The system according to claim 9, wherein the analysis algorithm corresponds to an event-detection algorithm implemented in the medical device (2).

11. The system according to one of the claims 9 or 10, wherein the clinical event comprises at least one of: QRS detection, P-wave detection, T-wave detection, atrial fibrillation detection, bradycardia detection, asystole detection, sudden rate drop detection, and high ventricular rate detection.

12. The system according to one of the claims 9 to 11, wherein the classification results are adjudicated as true or false.

13. The system according to claim 12, wherein the optimized parameter setting is selected such that false classifications are reduced.

14. The system according to one of claims 9 to 13, wherein the comparison comprises determining a performance metric for each parameter combination.

15. The system according to claim 14, wherein the performance metric comprises sensitivity and/or precision.
